(19) Europäisches Patentamt
European Patent Office
Office européen des brevets

(11) **EP 4 697 009 A1**

(12) **EUROPEAN PATENT APPLICATION**
published in accordance with Art. 153(4) EPC

(43) Date of publication:
**18.02.2026   Bulletin 2026/08**

(21) Application number: 23933150.7

(22) Date of filing: **31.05.2023**

(51) International Patent Classification (IPC):
*G01N 15/14* (2024.01)   *G01N 15/10* (2024.01)
*G01N 21/64* (2006.01)   *G01N 33/487* (2006.01)

(52) Cooperative Patent Classification (CPC):
**G01N 15/10; G01N 15/14; G01N 21/64;
G01N 33/487**

(86) International application number:
**PCT/KR2023/007464**

(87) International publication number:
**WO 2024/214862 (17.10.2024 Gazette 2024/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.04.2023   KR 20230047869**

(71) Applicant: **Bredis Healthcare Inc.
Guri-si Gyeonggi-do 11918 (KR)**

(72) Inventor: **HWANG, Hyundoo
Busan 48298 (KR)**

(74) Representative: **M. Zardi & Co S.A.
Via G. B. Pioda, 6
6900 Lugano (CH)**

(54) **METHOD FOR DETECTING ANALYTE PARTICLES PRESENT IN TRACE AMOUNTS IN FLUID SAMPLE**

(57) The present invention includes the steps of: causing a fluid sample containing an analyte to flow over a substrate having an array of microchambers formed on a surface thereof, with a first capture substance that specifically binds to the analyte immobilized in the microchambers; binding the analyte to the first capture substance in each microchamber of the array of microchambers; causing a second capture substance, which specifically binds to the analyte and binds to a signal-generating substance, to flow over the substrate to react the analyte with the second capture substance; causing the signal-generating substance to flow over the substrate to bind to the second capture substance; causing a substrate solution, which reacts with the signal-generating substance to generate a fluorescence signal, to flow over the substrate; causing a hydrophobic solvent to flow over the substrate to remove the substrate solution present outside the microchambers when the signal-generating substance and the substrate solution react in the microchambers; and counting and detecting the number of microchambers in which the fluorescence signal is generated.

[FIG. 1]

## Description

[Technical Field]

The present invention relates to a method for detecting particles of an analyte, and more particularly, to a method for detecting particles of an analyte present in trace amounts in a fluid sample, which allows for easier calculation of a concentration of molecules or particles present in trace amounts in the fluid sample.

[Background Art]

Most current techniques for quantifying molecules present at low concentrations in fluid sample employ amplification methods that increase the number of reporter molecules in order to provide a measurable signal. For example, a well-known method includes the enzyme-linked immunosorbent assay (ELISA) for amplifying antibody-based signals. An immuno-PCR technique that combines a gene amplification technique with an immunoassay technology based on antigen-antibody reactions is also available. The immuno-PCR method is capable of detecting ultratrace amounts of protein but requires a complicated assay process and may produce false-positive signals.

Korean Patent Application Publication No. KR10-2011-0024846 relates to a 'quantitative analysis device and method for biomolecules using magnetic nanoparticles', and discloses a configuration that improves the precision of quantitative analysis of biomolecules by including flux concentrators that are magnetized by an external magnetic field and concentrate magnetic flux in a gap region, a microchannel for injecting a sample solution into the gap region, and a sensor unit including a magnetic sensor formed on a substrate in the gap region between the flux concentrators to detect a change in the magnetic field in the gap region caused by magnetic nanoparticles.

However, in the related art, an output voltage of a GMR sensor changes by utilizing the fact that magnetic nanoparticles induce a change in the magnetic field in the gap region, a concentration of the magnetic nanoparticles is quantitatively analyzed through the magnitude of the change in the output voltage, and a target molecule is quantitatively analyzed. However, when the target molecule is present in trace amounts in a fluid sample, the magnitude of the change in the output voltage is too low to be measured. Therefore, there is a need to further increase the sensitivity of the quantitative analysis of the target molecule.

[Disclosure]

[Technical Problem]

Accordingly, the problem to be solved by the present invention is to provide a method for detecting particles of an analyte, which allows for easier calculation of a concentration of molecules or particles present in trace amounts in a fluid sample.

[Technical Solution]

To achieve the above object, the present invention provides a method for detecting an analyte present in trace amounts in a fluid sample, the method including: causing a fluid sample containing an analyte to flow over a substrate having an array of microchambers formed on a surface thereof, with a first capture substance that specifically binds to the analyte immobilized in the microchambers; binding the analyte to the first capture substance in each microchamber of the array of microchambers; causing a second capture substance, which specifically binds to the analyte and binds to a signal-generating substance, to flow over the substrate to react the analyte with the second capture substance; causing the signal-generating substance to flow over the substrate to bind to the second capture substance; causing a substrate solution, which reacts with the signal-generating substance to generate a fluorescence signal, to flow over the substrate; causing a hydrophobic solvent to flow over the substrate to remove the substrate solution present outside the microchambers when the signal-generating substance and the substrate solution react in the microchambers; and counting and detecting the number of microchambers in which the fluorescence signal is generated.

According to an exemplary embodiment of the present invention, preferably, the inside of the microchamber with the first capture substance immobilized therein is hydrophilic, and the outside of the microchamber is hydrophobic.

In addition, the method may further include calculating a first count value by counting the microchambers, calculating a second count value by recognizing and counting the microchambers in which a signal is generated due to mixing of the signal-generating substance and the substrate solution, and estimating an amount of the analyte in the fluid sample by calculating a ratio of the second count value to the first count value.

According to another exemplary embodiment of the present invention, preferably, the method may further include generating a calibration curve showing a concentration of a standard substance with a known concentration for the fluid sample and then correcting a concentration calculation of the analyte using the generated calibration curve.

According to another exemplary embodiment of the present invention, the method may further include estimating an amount of the analyte in the fluid sample by calculating a first count value by counting the microchambers, and calculating a second count value by capturing an image of a region of the microchambers, distinguishing the microchambers in which the fluorescence signal is generated through image processing, and counting the number of the distinguished microchambers.

[Advantageous Effects]

According to the present invention, the concentration of molecules or particles present in trace amounts in a fluid sample can be more easily calculated.

In addition, according to the present invention, the inside of the microchamber is coated with a hydrophilic solvent, and the outside of the microchamber is coated with a hydrophobic solvent, so that, when a hydrophobic solvent is caused to flow over the substrate, the substrate solution present outside the microchamber can be removed, thereby facilitating detection of the microchamber in which a fluorescence signal is generated.

[Description of Drawings]

FIG. 1 is a schematic diagram of a device for detecting particles of an analyte present in trace amounts in a fluid sample according to a first exemplary embodiment of the present invention.

FIG. 2 is a diagram illustrating in more detail an analysis chip 300 according to the first exemplary embodiment of the present invention.

FIG. 3 is a conceptual diagram illustrating a process for detecting an analyte according to the first exemplary embodiment of the present invention.

FIG. 4 is a flowchart of a method for detecting particles of an analyte present in trace amounts in a fluid sample according to the first exemplary embodiment of the present invention.

FIG. 5 is a schematic diagram of a device for detecting particles of an analyte present in trace amounts in a fluid sample according to a second exemplary embodiment of the present invention.

FIG. 6 is a diagram illustrating in more detail an analysis chip 301 according to the second exemplary embodiment of the present invention.

FIG. 7 is a diagram illustrating a combined form of a microparticle, a first capture substance, an analyte, and a signal-generating substance according to the second exemplary embodiment of the present invention.

FIG. 8 is a diagram illustrating in detail a state in which microparticles are captured in microchambers arranged in a detection space according to the second exemplary embodiment of the present invention.

FIG. 9 is a conceptual diagram illustrating a process for detecting an analyte according to the second exemplary embodiment of the present invention.

FIGS. 10 and 11 are diagrams illustrating a preprocessing process of a method for detecting particles of an analyte according to the second exemplary embodiment of the present invention.

FIG. 12 is a flowchart of a method for detecting particles of an analyte present in trace amounts in a fluid sample according to the second exemplary embodiment of the present invention.

FIG. 13 is a schematic diagram of a device for detecting particles of an analyte present in trace amounts in a fluid sample according to a third exemplary embodiment of the present invention.

FIG. 14 is a diagram illustrating in more detail an analysis chip 302 according to the third exemplary embodiment of the present invention.

FIG. 15 is a diagram illustrating in more detail a microwell microparticle according to the third exemplary embodiment of the present invention.

FIG. 16 is a conceptual diagram illustrating a process for detecting an analyte according to the third exemplary embodiment of the present invention.

FIG. 17 is a flowchart of a method for detecting particles of an analyte present in trace amounts in a fluid sample according to the third exemplary embodiment of the present invention.

[Best Mode]

The present invention relates to a method for detecting particles of an analyte present in trace amounts in a fluid sample, and is characterized by including the steps of: causing a fluid sample containing an analyte to flow over a substrate having an array of microchambers formed on a surface thereof, with a first capture substance that specifically binds to the analyte immobilized in the microchambers; binding the analyte to the first capture substance in each microchamber of the array of microchambers; causing a second capture substance, which specifically binds to the analyte and also binds to a signal-generating substance, to flow over the substrate to react the analyte with the second capture substance; causing the signal-generating substance to flow over the substrate to bind to the second capture substance; causing a substrate solution, which reacts with the signal-generating substance to generate a fluorescence signal, to flow over the substrate; causing a hydrophobic solvent to flow over the substrate to remove the substrate solution present outside the microchambers when the signal-generating substance and the substrate solution react in the microchambers; and counting and detecting the number of microchambers in which the fluorescence signal is generated.

[Best Mode]

Hereinafter, preferred exemplary embodiments of the present invention will be described in detail with reference to the accompanying drawings so that one skilled in the art to which the present invention pertains can readily implement the present invention. However, the exemplary embodiments are to describe in more detail the present invention, and it will be apparent to one skilled in the art that the scope of the present invention is not limited thereto.

Configurations of the present invention to elucidate solutions for the problems to be solved by the present invention will be described in detail with reference to the accompanying drawings, based on the preferred exemplary embodiments of the present invention. In attaching reference numerals to components of the drawings, the same components are denoted by the same reference numerals even though the components are present in other drawings, and in describing the drawings, it will be appreciated that components of other drawings may be cited if necessary. Further, in describing in detail an operation principle of preferred exemplary embodiments of the present invention, when a detailed description of well-known functions or components relating to the present invention and other matters may unnecessarily make unclear the gist of the present invention, the detailed description thereof will be omitted.

In addition, throughout the specification, when a portion is described as being "connected" to another portion, this includes not only being "directly connected" but also being "indirectly connected" with one or more intervening elements. Unless particularly stated otherwise in the present specification, a singular form also includes a plural form. The terms such as "comprises", "comprising", "includes", and/or "including", when used herein, do not exclude the presence or addition of one or more other components, steps, operations, or elements other than the stated components, steps, operations, or elements.

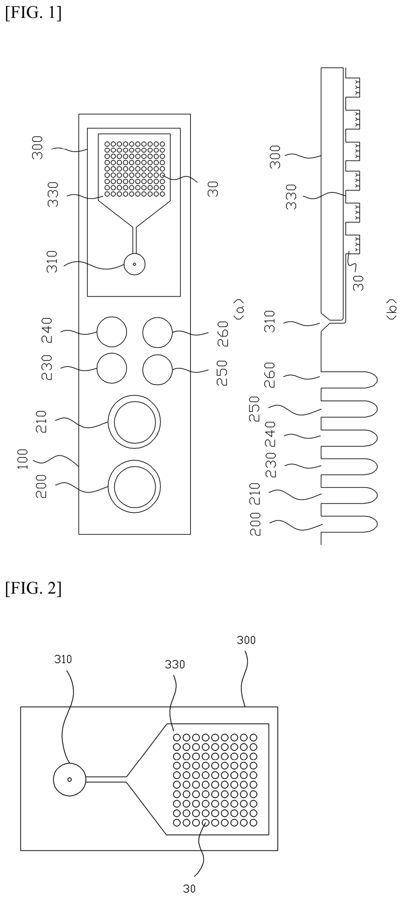

FIG. 1 is a schematic diagram of a device for detecting particles of an analyte present in trace amounts in a fluid sample according to a first exemplary embodiment of the present invention.

FIG. 1(a) is a plan view of a device for detecting particles of an analyte according to a first exemplary embodiment of the present invention, and FIG. 1(b) is a cross-sectional view of the device for detecting particles of an analyte according to the first exemplary embodiment of the present invention.

Referring to FIG. 1, the device for detecting particles of an analyte present in trace amounts in a fluid sample according to the first exemplary embodiment of the present invention includes a cartridge 100.

The cartridge 100 includes a fluid sample chamber 200, a waste chamber 210, a washing solution chamber 230, a second capture substance chamber 240, a signal-generating substance chamber 250, a substrate solution chamber 260, and an analysis chip 300.

An upper surface of the cartridge 100 is preferably sealed with an air-impermeable film using thermal welding, UV bonding, ultrasonic welding, tape, or the like in order to prevent contamination between the plurality of chambers and the analysis chip 300 or contamination from external substances.

The fluid sample chamber 200 is configured to accommodate a predetermined amount of fluid sample. The fluid sample chamber 200 may be configured to have a capacity within a range of 50 μL to 10 mL. The fluid sample includes body fluids such as blood, plasma, serum, and cerebrospinal fluid.

The waste chamber 210 is configured to provide a storage space for accommodating waste that is generated when a fluid sample, a washing solution, a second capture substance, a signal-generating substance, a substrate solution, and the like introduced into the analysis chip 300 are discharged. The waste chamber 210 may be configured to have a capacity of 1 to 15 mL. The waste chamber 210 of FIG. 1 is included in the cartridge 100. However, the waste chamber 210 may be provided in a separate space outside the cartridge 100.

The washing solution chamber 230 is configured to accommodate a washing solution for washing a detection space 330 in which microchambers 30 of the analysis chip 300 are provided. The washing solution may be a solution such as phosphate buffered saline (PBS), tris-buffered saline (TBS), PBS-T, TBS-T, or ultrapure water. As an example, the washing solution of the washing solution chamber 230 may be injected into an injection port 310 to wash away the fluid sample, the second capture substance, the signal-generating substance, the substrate solution, and the like remaining in the detection space 330 after reaction.

The second capture substance chamber 240 is configured to accommodate a second capture substance that specifically binds to an analyte and also binds to a signal-generating substance. The second capture substance has the characteristic of specifically binding to each of the analyte and the signal-generating substance, and functions to cause the signal-generating substance to bind to the second capture substance that has specifically bound to the analyte, thereby generating a fluorescence signal proportional to the amount of the analyte. For example, the second capture substance may be a secondary antibody and may be provided in a form pre-bound to a signal-generating substance.

Since the second capture substance binds to the analyte, and the first capture substance immobilized in a plurality of microchambers 30 also binds to the analyte, it is preferable that the first capture substance and the second capture substance bind to different sites of the analyte.

The signal-generating substance chamber 250 is configured to accommodate a signal-generating substance that specifically binds to the second capture substance bound to the analyte. The signal-generating substance may be, for example, an enzyme.

The signal-generating substance is, for example, a chromogenic, fluorescent, or chemiluminescent enzyme precursor, which is converted into a detectable label upon contact with a substrate solution.

The signal-generating substance and the second capture substance may be accommodated in a bound state in either the second capture substance chamber 240 or the signal-generating substance chamber 250.

The signal-generating substance serves to generate a signal of a detectable indicator, such as a fluorescence signal, by reacting with a substrate solution, and the enzyme component may include β-galactosidase, horseradish peroxidase, or alkaline phosphatase.

The substrate solution chamber 260 is configured to accommodate a substrate solution that reacts with the signal-generating substance to generate a signal. The substrate solution reacts with the signal-generating substance to generate a signal of a detectable indicator, such as a fluorescence signal, and may include Resorufin β-D-galactopyranoside, or the like.

The analysis chip 300 includes the injection port 310 and the detection space 330.

The fluid sample, the washing solution, the second capture substance, the signal-generating substance, the substrate solution, or a mixture thereof is injected through the injection port 310.

The injection port 310 may further include a filter to separate and supply plasma from whole blood. The filter filters out blood cells in the whole blood and allows the fluid sample containing plasma components to pass through. The filter may be formed of various materials with pores of various sizes to filter out biological cells, inorganic particles, organic particles, or the like. The filter of the present exemplary embodiment preferably has pores capable of filtering out cells or foreign substances larger than the analyte, and is preferably formed with pores capable of filtering out blood cells having a size of approximately 5 to 10 μm. Additionally, the filter may be formed of a biologically inert material so that it can be applied to biological samples.

In the process of supplying the fluid sample to the injection port 310, an output surface of the filter may be pre-wetted by wetting it with a buffer solution before filtration. Therefore, the plasma of whole blood supplied to an inlet surface of the filter can pass through the filter pores without surface resistance and be smoothly filtered to the outlet surface of the filter.

The detection space 330 has a plurality of microchambers 30 arranged therein, and a plurality of first capture substances are preferably immobilized inside each microchamber.

The microchambers 30 are present in an array form on one surface of the detection space 330 of the analysis chip 300 at regular intervals from each other, and the size of each microchamber is the same. The number of microchambers is preferably 10,000 or more, and the number may vary depending on the concentration range of the analyte to be measured. A first capture substance that specifically binds to the analyte is immobilized on an inner surface of the microchamber 30. Additionally, to prevent non-specific reactions, the remaining surface where the first capture substance is absent is preferably coated with a blocking substance such as bovine serum albumin (BSA).

When a fluid sample is injected through the injection port 310, the analyte contained in the fluid sample combine binds to the first capture substance immobilized in the inner space of the microchamber 30 within the detection space 330.

Thereafter, when a washing solution is injected through the injection port 310, the remaining fluid sample after the binding of the first capture substance and the analyte can be removed, and washing can be performed.

Note that, when the second capture substance accommodated in the second capture substance chamber 240 that binds to the analyte and the signal-generating substance is injected through the injection port 310, the second capture substance binds to the analyte bound to the first capture substance. Subsequently, the remaining second capture substance can be removed, and washing can be performed by the washing solution injected through the injection port 310.

Next, when a signal-generating substance is injected through the injection port 310, the signal-generating substance binds to the second capture substance.

Finally, a substrate solution is injected through the injection port 310 to cause the substrate solution to react with the signal-generating substance, thereby generating a fluorescence signal, and a hydrophobic solvent is caused to flow through the injection port 310 to remove the remaining substrate solution after the reaction with the signal-generating substance. The hydrophobic solvent is preferably oil or gas.

FIG. 2 is a diagram illustrating in more detail an analysis chip 300 according to the first exemplary embodiment of the present invention.

The fluid sample, the washing solution, the second capture substance, the signal-generating substance, and the substrate solution, or a mixed solution thereof are successively injected through the injection port 310. The second capture substance is a capture substance for binding both an analyte and a signal-generating substance that generates a signal of a detectable indicator, such as a fluorescence signal, and may be provided with bound to the signal-generating substance, which can simplify the procedure compared to when the second capture substance and the signal-generating substance are provided separately.

When the fluid sample, the washing solution, the second capture substance, the signal-generating substance, and the substrate solution or a mixed solution thereof are injected through the injection port 310, they diffuse and flow into the detection space 330 and flow over the microchambers 30.

It is preferable that the first capture substance, which binds to the analyte contained in the fluid sample, be immobilized in the microchambers 30.

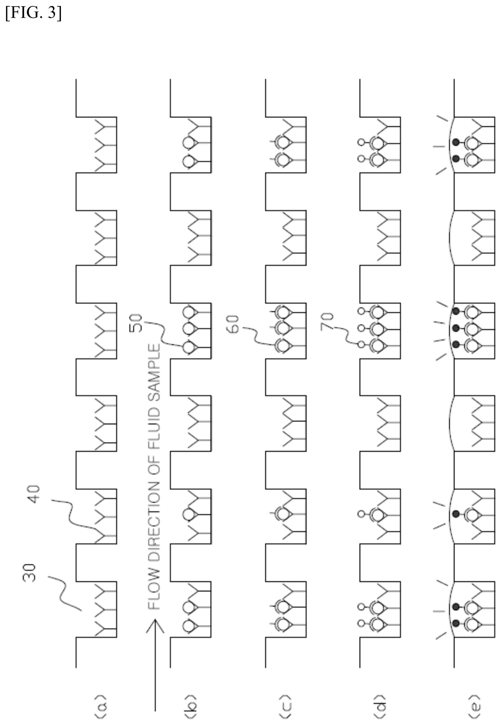

FIG. 3 is a conceptual diagram illustrating a process for detecting an analyte according to the first exemplary embodiment of the present invention.

FIG. 3(a) illustrates the microchambers 30 concavely formed in a lower portion of the substrate, with a first capture substance 40 immobilized in the microchambers 30. A size of each of the microchambers 30 is 0.5 to 15 μm, which is the minimum size that allows for optical measurement.

The microchambers 30 are formed in an array along a bottom surface of the detection space 330 with a spacing of at least 0.5 μm from each other, and may be designed so that at least 10,000 microchambers are arranged in a uniform distribution.

The microchamber 30 is formed as a groove in the bottom surface of the detection space 330, and the first capture substance 40 is immobilized to specifically capture an analyte 50.

In an exemplary embodiment of the present invention, the microchambers 30 may be formed in a direction perpendicular to a fluid flow direction on the bottom surface of the detection space 330. Accordingly, the microchambers 30 and the fluid flow are arranged at right angles. Therefore, the particles inserted into the microchambers 30 cannot easily escape from the microchambers 30 along the fluid flow.

In this way, the first capture substance 40 is stably inserted into and immobilized in the microchamber 30, maintaining a uniform array in the detection space 330 regardless of the fluid flow.

When the first capture substance is an antibody particle, the size of the antibody is generally 10 to 50 nm, so it is preferable that a large number of antibodies be immobilized in the microchamber 30.

FIG. 3(b) illustrates that the analyte 50 is captured by the first capture substance 40 as the fluid sample flows over the microchambers 30. It is preferable that the remaining fluid sample containing the uncaptured analyte be washed by a washing solution.

As can be seen in FIG. 3(b), since a plurality of first capture substances are immobilized in the microchamber 30, the number of analytes 50 immobilized on the first capture substances within one microchamber 30 may vary depending on the number and properties of the analytes, such that either one analyte is captured per microchamber, or the number of captured analytes differs from microchamber to microchamber. For example, when the number of analytes is less than 60% of the number of microchambers, there is a high probability that one analyte is captured in each microchamber. However, when the number of analytes is equal to or greater than 60% of the number of microchambers, the number of analytes captured in each microchamber may differ from microchamber to microchamber. Therefore, when the number of analytes captured in each microchamber is the same, it is preferable to estimate the number of analytes by calculating the number of microchambers in which a signal of a detectable indicator, such as a fluorescence signal, is generated. When the number of analytes captured in each microchamber is different, it is preferable to estimate the number of analytes by calculating not only the number of microchambers in which a signal of a detectable indicator, such as a fluorescence signal, is generated, but also the intensity of the signal of the detectable indicator, such as a fluorescence signal, generated in the microchambers.

The remaining substances, other than the analytes inserted and immobilized or captured in the microchambers 30, are removed from the detection space 330 by the washing solution. During the washing process, since the substances inserted and immobilized or captured in the microchambers are inserted and immobilized in the microchambers 30 formed deep in a direction perpendicular to the flow direction of the washing solution, they can remain immobilized without escaping from the microchambers 30 by the flow of the washing solution. Accordingly, only the remaining substances that are not bound to and immobilized by the first capture substance inserted and immobilized in the microchambers 30 within the detection space 330 flow out from the detection space 330 along the flow of the washing solution and are removed.

FIG. 3(c) illustrates a state in which, when a second capture substance 60 is allowed to flow over the microchambers 30, the second capture substance 60 binds to and reacts with the analyte 50. It is preferable that the remaining second capture substance 60 be then washed away by a washing solution.

FIG. 3d illustrates that, when a signal-generating substance 70 is caused to flow over the microchambers 30, the signal-generating substance 70 binds to and reacts with the second capture substance 60. It is preferable that the remaining signal-generating substance 70 be then washed away by a washing solution.

FIG. 3(e) illustrates that a substrate solution is caused to flow over the microchambers 30, and the substrate solution reacts with the signal-generating substance 70 to generate a signal of a detectable indicator, such as a fluorescence signal. It is preferable that the remaining substrate solution be then washed away by a washing solution.

In FIG. 3(b), since the number of analytes 50 immobilized on the first capture substance in one microchamber 30 differs, the number of signal-generating substances 70 that generate a signal of a detectable indicator, such as a fluorescence signal, also differs from microchamber to microchamber.

Thereafter, a hydrophobic solvent is caused to flow over the substrate to remove the substrate solution present outside the plurality of microchambers 30, and the number of microchambers where the fluorescence signal is generated is counted and detected. The inside of the plurality of microchambers 30 is coated with a hydrophilic solvent, and the outside of the plurality of microchambers 30 is coated with a hydrophobic solvent, so that, when the hydrophobic solvent is caused to flow over the substrate, the substrate solution present outside the plurality of microchambers 30 can be removed.

For more accurate detection of the analyte contained in the fluid sample, it is preferable to correlate the number of particles of the analyte with the brightness of a signal of a detectable indicator, such as a fluorescence signal, and to count the number of particles of the analyte by considering the brightness of the signal of the detectable indicator, such as a fluorescence signal, in each microchamber.

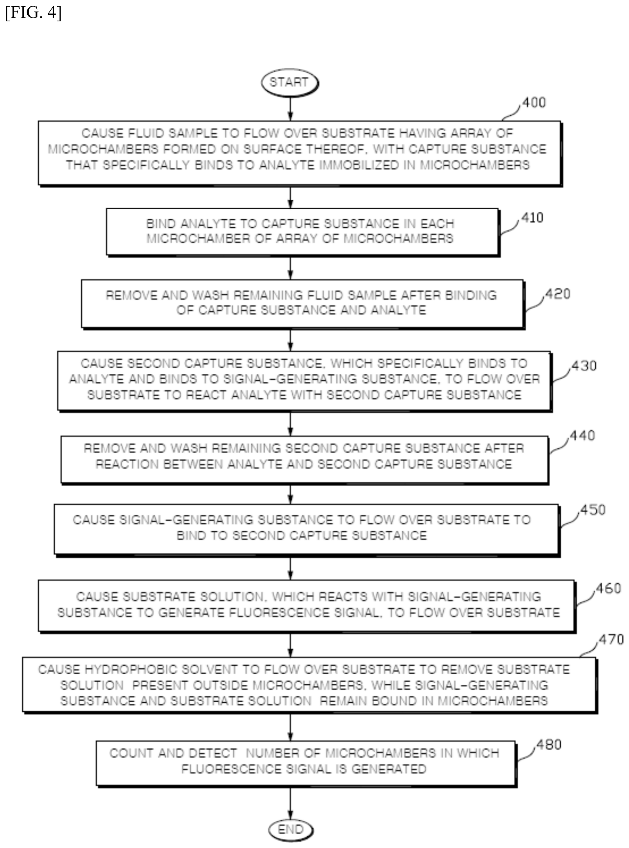

FIG. 4 is a flowchart of a method for detecting particles of an analyte present in trace amounts in a fluid sample according to the first exemplary embodiment of the present invention.

In step 400, a fluid sample containing an analyte is caused to flow over a substrate having an array of microchambers formed on a surface thereof, with a first capture substance that specifically binds to the analyte immobilized in the microchambers.

In step 410, the analyte binds to the first capture substance in each microchamber of the array of microchambers 30.

In step 420, the remaining fluid sample after the binding of the first capture substance and the analyte is removed, and washing can be performed.

In step 430, a second capture substance, which specifically binds to the analyte and binds to a signal-generating substance, is caused to flow over the substrate to react the analyte with the second capture substance.

In step 440, the remaining second capture substance after the reaction between the analyte and the second capture substance is removed, and washing can be performed.

In step 450, the signal-generating substance is caused to flow over the substrate to bind to the second capture substance.

In step 460, a substrate solution, which reacts with the signal-generating substance to generate a signal of a detectable indicator, such as a fluorescence signal, is caused to flow over the substrate.

In step 470, a hydrophobic solvent is caused to flow over the substrate to remove the substrate solution present outside the microchambers, while the signal-generating substance and the substrate solution remain bound in the microchambers.

It is preferable that the inside of the microchamber be hydrophilic and the outside of the microchamber be hydrophobic.

In step 480, the number of microchambers in which the signal of the detectable indicator, such as the fluorescence signal, is generated is counted and detected.

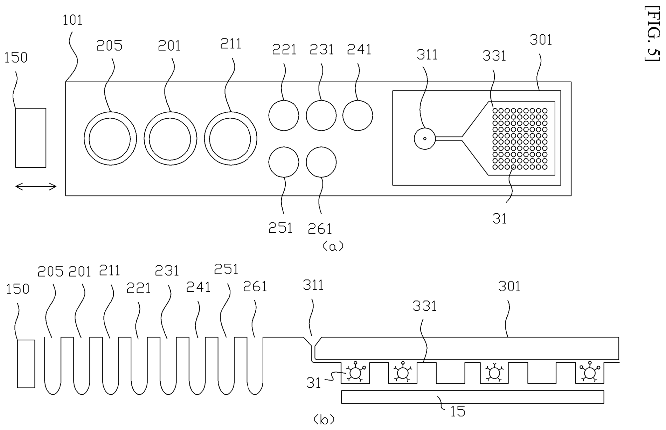

FIG. 5 is a schematic diagram of a device for detecting particles of an analyte present in trace amounts in a fluid sample according to a second exemplary embodiment of the present invention.

FIG. 5(a) is a plan view of a device for detecting particles of an analyte according to a second exemplary embodiment of the present invention, and FIG. 5(b) is a cross-sectional view of the device for detecting particles of an analyte according to the second exemplary embodiment of the present invention.

Referring to FIG. 5, the device for detecting particles of an analyte present in trace amounts in a fluid sample according to the second exemplary embodiment of the present invention includes a magnetic device 150 and a cartridge 101.

The magnetic device 150 moves closer to or farther away from a reaction chamber 205, thereby causing separation between magnetic particles, which are an example of microparticles, within the reaction chamber 205, and a residual solution. Thereafter, after removing the residual solution, a washing solution accommodated in a washing solution chamber 231 may be injected into the reaction chamber 205 to wash a wall surface of the reaction chamber 205 where the magnetic particles are located.

The cartridge 101 includes a fluid sample chamber 201, the reaction chamber 205, a waste chamber 211, a microparticle chamber 221, the washing solution chamber 231, a second capture substance chamber 241, a signal-generating substance chamber 251, a substrate solution chamber 261, and an analysis chip 301.

An upper surface of the cartridge 101 is preferably sealed with an air-impermeable film using thermal welding, UV bonding, ultrasonic welding, tape, or the like in order to prevent contamination between the plurality of chambers and the analysis chip 301 or contamination from external substances.

The fluid sample chamber 201 is configured to accommodate a predetermined amount of fluid sample. The fluid sample chamber 201 may be configured to have a capacity within a range of 50 μL to 10 mL. The fluid sample includes body fluids such as blood, plasma, serum, and cerebrospinal fluid.

The reaction chamber 205 is configured to accommodate and mix a predetermined amount of a fluid sample and microparticles with a first capture substance immobilized thereon. The reaction chamber 205 may be configured to have a capacity within a range of 50 μL to 15 mL.

The waste chamber 211 is configured to provide a storage space for accommodating waste that is generated when a fluid sample, microparticles, a washing solution, a second capture substance, a signal-generating substance, a substrate solution, and the like introduced into the analysis chip 301 are discharged. The waste chamber 211 may be configured to have a capacity within a range of 1 to 30 mL. The waste chamber 211 of FIG. 5 is included in the cartridge 101. However, the waste chamber 211 may be provided in a separate space outside the cartridge 101.

The microparticle chamber 221 is configured to accommodate microparticles with a first capture substance immobilized thereon, and the microparticles are injected into the reaction chamber 205. The microparticles may be polymer or magnetic particles. The magnetic particles have a diameter of between 500 nm and 10 μm, and preferably contain a magnetic material such as iron oxide. The microparticles may be spherical magnetic particles or donut-shaped microchamber particles.

It is preferable that the microparticles accommodated in the microparticle chamber 221 have a first capture substance bound to their surfaces, the first capture substance binding to an analyte. The first capture substance may include an antibody, a nucleic acid molecule, a peptide, or the like.

The washing solution chamber 231 is configured to accommodate a washing solution for washing a wall surface of the reaction chamber 205 and a detection space 331 in which microchambers 31 of the analysis chip 301 are provided. The washing solution may be a solution such as phosphate buffered saline (PBS), tris-buffered saline (TBS), PBS-T, TBS-T, or ultrapure water.

As an example, the washing solution of the washing solution chamber 231 may be injected into an injection port 311 to wash away the fluid sample, the microparticles, the second capture substance, the signal-generating substance, the substrate solution, and the like remaining in the detection space 331 after reaction.

In another exemplary embodiment, when the magnetic device 150 comes close to the reaction chamber 205 and separation occurs between the magnetic particles, which are an example of microparticles, within the reaction chamber 205, and the residual solution, after removing the residual solution, the washing solution accommodated in the washing solution chamber 231 may be injected into the reaction chamber 205 to wash the wall surface of the reaction chamber 205 where the magnetic particles are located.

The second capture substance chamber 241 is configured to accommodate a second capture substance that specifically binds to an analyte and also binds to a signal-generating substance. The second capture substance has the characteristic of specifically binding to each of the analyte and the signal-generating substance, and functions to cause the signal-generating substance to bind to the second capture substance that has specifically bound to the analyte, thereby generating a fluorescence signal proportional to the amount of the analyte. The second capture substance may be, for example, a secondary antibody and may be provided in a form pre-bound to the signal-generating substance.

Since the second capture substance binds to the analyte, and the first capture substance immobilized on the microparticles also binds to the analyte, it is preferable that the first capture substance and the second capture substance bind to different sites of the analyte.

The signal-generating substance chamber 251 is configured to accommodate a signal-generating substance that specifically binds to the second capture substance bound to the analyte. The signal-generating substance may be, for example, an enzyme.

The signal-generating substance is, for example, a chromogenic, fluorescent, or chemiluminescent enzyme precursor, which is converted into a detectable label upon contact with a substrate solution.

The signal-generating substance and the second capture substance may be accommodated in a bound state in either the second capture substance chamber 241 or the signal-generating substance chamber 251.

The signal-generating substance serves to generate a signal of a detectable indicator, such as a fluorescence signal, by reacting with a substrate solution, and the enzyme component may include β-galactosidase, horseradish peroxidase, or alkaline phosphatase.

The substrate solution chamber 261 is configured to accommodate a substrate solution that reacts with the signal-generating substance to generate a signal. The substrate solution reacts with the signal-generating substance to generate a signal of a detectable indicator, such as a fluorescence signal, and may include Resorufin β-D-galactopyranoside, or the like.

The analysis chip 301 includes the injection port 311 and the detection space 331.

Since a predetermined amount of fluid sample and the microparticles with the first capture substance immobilized thereon are accommodated and mixed in the reaction chamber 205, the analyte is bound to the first capture substance immobilized on the microparticles.

The fluid sample, the microparticles, the washing solution, the second capture substance, the signal-generating substance, and the substrate solution, or a mixture is injected through the injection port 311.

The injection port 311 may further include a filter to separate and supply particles larger than microparticles from a mixed solution of the microparticles and the fluid sample. The filter may be formed of various materials with pores of various sizes to filter out biological cells, inorganic particles, organic particles, or the like. It is preferable that the filter of the second exemplary embodiment be formed with pores capable of filtering out particles or foreign substances larger than microparticles. Additionally, the filter may be formed of a biologically inert material so that it can be applied to biological samples.

In the process of supplying the fluid sample to the injection port 311, an output surface of the filter may be pre-wetted by wetting it with a buffer solution before filtration. Therefore, the mixed solution of microparticles and fluid sample supplied to an inlet surface of the filter can pass through the filter pores without surface resistance and be smoothly filtered to the outlet surface of the filter.

The detection space 331 has a plurality of microchambers 31 arranged therein, and when the mixed solution of microparticles and fluid sample is injected through the injection port 311, the microparticles are captured in the inner spaces of the microchambers 31 within the detection space 331. By placing a magnetic body 15 under the microchambers 31, microparticles, if the microparticles are magnetic particles, can be easily captured in the inner spaces of the microchambers 31.

The microparticles captured in the microchambers 31 are a mix of microparticles where the analyte is captured by the first capture substance and microparticles where it is not captured. The size of the microchamber 31 is preferably larger than the diameter of the microparticles and smaller than twice the diameter of the microparticles.

Thereafter, when a washing solution is injected through the injection port 311, the remaining fluid sample after the binding of the first capture substance and the analyte can be removed, and washing can be performed.

Note that, when the second capture substance accommodated in the second capture substance chamber 241 that binds to the analyte and the signal-generating substance is injected through the injection port 311, the second capture substance binds to the analyte bound to the first capture substance. Subsequently, the remaining second capture substance can be removed, and washing can be performed by the washing solution.

Next, when a signal-generating substance is injected through the injection port 311, the signal-generating substance binds to the second capture substance.

Finally, a substrate solution is injected through the injection port 311 to cause the substrate solution to react with the signal-generating substance, thereby generating a signal of a detectable indicator, such as a fluorescence signal, and a hydrophobic solvent is caused to flow through the injection port 311 to remove the remaining substrate solution after the reaction with the signal-generating substance. The hydrophobic solvent is preferably oil or gas.

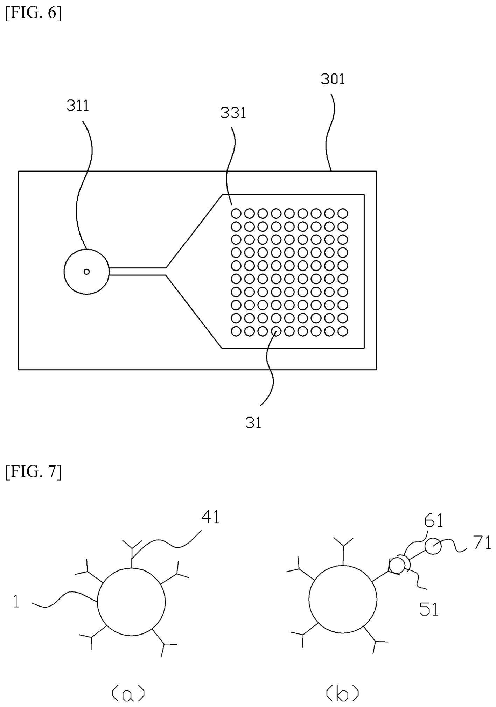

FIG. 6 is a diagram illustrating in more detail an analysis chip 301 according to the second exemplary embodiment of the present invention.

The mixed solution of fluid sample and microparticles, the washing solution, the second capture substance, the signal-generating substance, and the substrate solution, or a mixed solution thereof are successively injected through the injection port 311. The second capture substance is a capture substance for binding both an analyte and a signal-generating substance that generates a signal of a detectable indicator, such as a fluorescence signal, and may be provided with bound to the signal-generating substance, which can simplify the procedure compared to when the second capture substance and the signal-generating substance are provided separately.

When the mixed solution of fluid sample and microparticles, the washing solution, the second capture substance, the signal-generating substance, and the substrate solution or a mixed solution thereof are injected through the injection port 311, they diffuse and flow into the detection space 331 and flow over the microchambers 31.

The size of the microchamber 310 is preferably larger than the diameter of the microparticle and smaller than twice the diameter of the microparticle.

FIG. 7 is a diagram illustrating a combined form of a microparticle, a first capture substance, an analyte, and a signal-generating substance according to the second exemplary embodiment of the present invention.

Referring to FIG. 7(a), it illustrates that a first capture substance 41 is immobilized on a microparticle 1, and referring to FIG. 7(b), it illustrates that an analyte 51 is bound to the first capture substance 41, a second capture substance 61 is bound to the analyte 51, and a signal-generating substance 71 is bound to the second capture substance 61. When the substrate solution reacts with the signal-generating substance 71, a signal of a detectable indicator, such as a fluorescence signal, is generated.

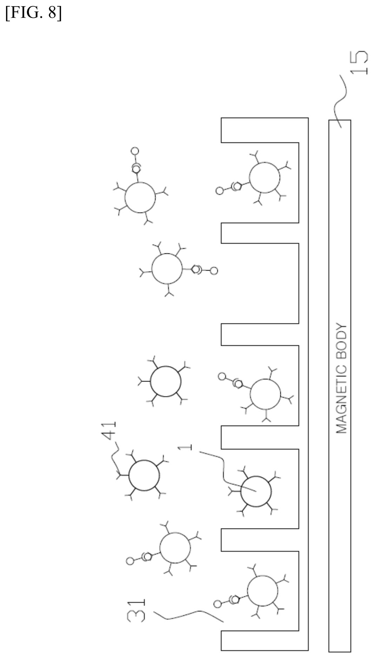

FIG. 8 is a diagram illustrating in detail a state in which microparticles are captured in microchambers arranged in a detection space according to the second exemplary embodiment of the present invention.

Referring to FIG. 8, it illustrates a state in which the magnetic body 15 is placed below the inner spaces of the microchambers 31, thereby forming a magnetic field, and capturing magnetic particles as an example of the microparticles 1 in the mixed solution.

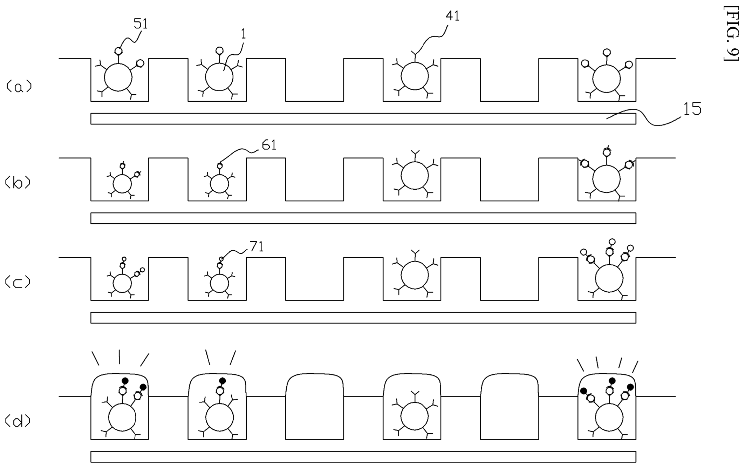

FIG. 9 is a conceptual diagram illustrating a process for detecting an analyte according to the second exemplary embodiment of the present invention.

FIG. 9(a) illustrates a state in which the magnetic particles 1 with the first capture substance 41 immobilized thereon, after being mixed and reacted with a fluid sample in the reaction chamber 205 and injected through the injection port 311, are captured in the respective microchambers 31 by the magnetic body 15. The first capture substance 41 is immobilized on the magnetic particle 1.

As the mixed solution coming out of the injection port 311 flows over the plurality of microchambers 31, the magnetic particles are captured in each receiving portion by the magnetic body placed below the plurality of microchambers 31.

The size of the microchamber 31 is preferably larger than the diameter of the magnetic particle 1 and smaller than twice the diameter of the magnetic particle. Therefore, the receiving portion, which is an inner space of the microchamber 31, is larger than the size of the magnetic particle 1 and has a size that allows at most one magnetic particle to be accommodated.

The microchambers 31 are formed in an array at intervals along a bottom surface of the detection space 331, and may be designed to be arranged in a uniform distribution.

In an exemplary embodiment of the present invention, a depth of the microchamber 31 may be $d < h < 2d$. Here, h is the depth of the microchamber 31 and d is the diameter of the microparticle. The depth h of the microchamber 31 is a distance from the bottom surface of the microchamber 31 to the inner upper edge of the microchamber 31. Additionally, a width of the microchamber 31 may be $d < w < 2d$. Here, w is the width of the microchamber 30, and d is the diameter of the microparticle.

If the depth h of the microchamber 31 is smaller than the diameter d of the microparticle, the microparticle is not stably immobilized, which may cause the microparticle to escape from the microchamber 31 due to the flow of the fluid sample flowing along the detection space 331. If the depth h of the microchamber 31 exceeds twice the diameter d of the microparticle, two or more microparticles may be captured in one chamber, thereby lowering analysis accuracy, and the diffusion of the washing solution for washing the microparticles and the detection space 331 may take a long time.

Further, in an exemplary embodiment of the present invention, the microchambers 31 may be formed in a direction perpendicular to a fluid flow direction on the bottom surface of the detection space 331. Accordingly, the microchambers 31 and the fluid flow are arranged at right angles. Therefore, the microparticles inserted into the microchambers 31 cannot easily escape from the microchambers 31 along the fluid flow.

In this way, the microparticles are stably inserted in the microchambers 31, maintaining a uniform array in the detection space 331 regardless of the fluid flow. When the washing solution is injected through the injection port 311, the fluid sample containing the uncaptured analyte after binding of the first capture substance 41 and the analyte 51 can be removed, and washing can be performed. The magnetic particles 1 are not washed away because they are immobilized in the respective receiving portions of the microchambers 31 by the magnetic body.

The remaining substances, other than the magnetic particles inserted and immobilized or captured in the microchambers 31, are removed from the detection space 331 by the washing solution. During the washing process, since the substances inserted and immobilized or captured in the microchambers are inserted in the microchambers 31 formed deep in a direction perpendicular to the flow direction of the washing solution, they can remain immobilized without escaping from the microchambers 31 by the flow of the washing solution. Accordingly, only the remaining substances that are inserted into the microchambers 31 within the detection space 331 but not bound to and immobilized by the analyte flow out from the detection space 331 along the flow of the washing solution and are removed.

As can be seen in FIG. 9(b), when the second capture substance 61 accommodated in the second capture substance chamber 241 that binds to the analyte and the signal-generating substance is injected through the injection port 311, the second capture substance 61 binds to the analyte 51 bound to the first capture substance 41. Subsequently, the remaining second capture substance 61 can be removed, and washing can be performed by the washing solution.

In FIG. 9(c), when the signal-generating substance 71 is injected through the injection port 311, the signal-generating substance 71 binds to the second capture substance 61. It is preferable that the remaining signal-generating substance 71 be then washed away by a washing solution.

In FIG. 9(d), a substrate solution is injected through the injection port 311 to cause the substrate solution to react with the signal-generating substance 71, thereby generating a signal of a detectable indicator, such as a fluorescence signal, and a hydrophobic solvent is caused to flow through the injection port 311 to remove the remaining substrate solution after the reaction with the signal-generating substance 71.

Then, a hydrophobic solvent is caused to flow over the substrate to remove the substrate solution present outside the plurality of microchambers 31, and the number of microchambers where a signal of a detectable indicator, such as a fluorescence signal, is generated is counted and detected. The inside of the plurality of microchambers 31 is coated with a hydrophilic solvent, and the outside of the plurality of microchambers 31 is coated with a hydrophobic solvent, so that, when the hydrophobic solvent is caused to flow over the substrate, the substrate solution present outside the plurality of microchambers 31 can be easily removed.

For more accurate detection of the analyte contained in the fluid sample, the number of particles of the analyte may be correlated with the brightness of a signal of a detectable indicator, such as a fluorescence signal, and the number of particles of the analyte may be counted by considering the brightness of the signal of the detectable indicator, such as a fluorescence signal, in each microchamber.

Referring again to FIG. 9(d), it can be seen that the number of signal-generating substances that generate a signal of a detectable indicator, such as a fluorescence signal, differs from microchamber to microchamber, and the brightness of the fluorescence signal is different accordingly.

Therefore, when the number of signal-generating substances captured in each microchamber is the same, it is preferable to estimate the number of analytes by calculating the number of microchambers in which a fluorescence signal is generated. When the number of signal-generating substances captured in each microchamber is different, it is preferable to estimate the number of analytes by calculating not only the number of microchambers in which a fluorescence signal is generated, but also the intensity of the fluorescence signal generated in the microchambers.

According to another exemplary embodiment of the present invention, a first capture substance immobilized on microparticles and an analyte in a fluid sample may be reacted within the reaction chamber 200, the magnetic device may be brought close to the reaction chamber 200 to separate the microparticles from the fluid sample, and then the fluid sample not bound to the microparticle may be removed, and washing can be performed.
Thereafter, a second capture substance that specifically binds to the analyte bound to the first capture substance may be injected into and reacted in the reaction chamber 200, and a signal-generating substance that specifically binds to the second capture substance may be injected into and reacted in the reaction chamber 200.

Next, a substrate solution that reacts with the signal-generating substance to generate a signal is injected into and mixed in the reaction chamber 205, and magnetic particles in the mixed substrate solution are captured in the microchambers 31 within the detection space 331 using the magnetic body 15.

Finally, it is preferable that a device for detecting particles of the analyte perform detection by counting the number of microchambers 31 in which a signal is generated due to the mixing of the signal-generating substance and the substrate solution.

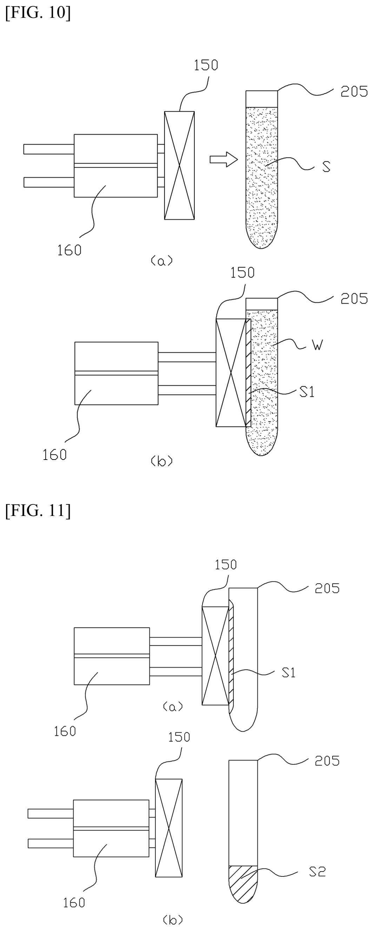

FIGS. 10 and 11 are diagrams illustrating a preprocessing process of a method for detecting particles of an analyte according to the second exemplary embodiment of the present invention.

First, as illustrated in FIG. 10(a), a magnetic device driving unit 160 may move the magnetic device 150 into contact with the reaction chamber 205 for 20 minutes. The reaction chamber 205 accommodates a mixed solution (S) in which a predetermined amount of a fluid sample and microparticles with the first capture substance immobilized thereon are mixed.

As a result, as illustrated in FIG. 10(b), separation between the magnetic particles S1 and the residual solution W may occur in the reaction chamber 205.

Next, as illustrated in FIG. 11(a), the residual solution W is removed from the reaction chamber 205, and the washing solution accommodated in the washing solution chamber 231 is injected into the reaction chamber 205, thereby washing the wall surface of the reaction chamber 205 where the magnetic particles S1 are located. Thereafter, the magnetic device driving unit 160 may move the magnetic device 150 again to separate the magnetic device 150 from the reaction chamber 205.

Next, as illustrated in FIG. 11(a), the magnetic particles S1 remain in the reaction chamber 205, and as illustrated in FIG. 11(b), it is preferable to prepare a mixed solution S2 with a separate buffer solution added thereto for injection into the injection port 311.

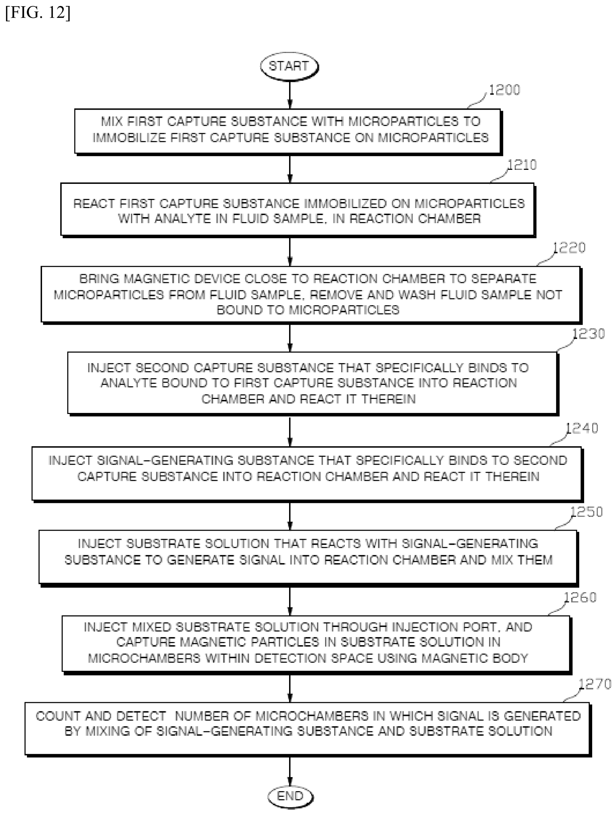

FIG. 12 is a flowchart of a method for detecting particles of an analyte present in trace amounts in a fluid sample according to the second exemplary embodiment of the present invention.

In step 1200, a first capture substance is mixed with microparticles so that the first capture substance is immobilized on the microparticles. The first capture substance may include an antibody, a nucleic acid molecule, a peptide, or the like. The microparticles are preferably magnetic particles.

The magnetic particles have a diameter of between 500 nm and 10 $\mu$m, and preferably contain a magnetic material such as iron oxide. According to an exemplary embodiment of the present invention, the mixing may be performed using a method such as vibration, ultrasound, or pipetting.

In step 1210, the first capture substance immobilized on the microparticles is reacted with an analyte in a fluid sample in the reaction chamber 200.

The fluid sample includes body fluids such as blood, plasma, serum, and cerebrospinal fluid.

In step 1220, the magnetic device is brought close to the reaction chamber 200 to separate the microparticles from the fluid sample, and the fluid sample not bound to the microparticles is removed, and washing can be performed.

After removing the fluid sample not bound to the microparticles, a washing solution is injected and mixed with the microparticles, then the magnetic body is brought close to the reaction chamber 200 to separate the magnetic particles from the washing solution, and the washing solution is removed, thereby performing washing of the microparticles.

In step 1230, a second capture substance that specifically binds to the analyte bound to the first capture substance is injected into and reacted in the reaction chamber 200. The second capture substance not bound to the analyte is removed, and washing can be performed.

Since the second capture substance binds to the analyte, and the first capture substance immobilized on the microparticles also binds to the analyte, it is preferable that the first capture substance and the second capture substance bind to different sites of the analyte.

In step 1240, a signal-generating substance that specifically binds to the second capture substance is injected into and reacted in the reaction chamber 200. The signal-generating substance not bound to the second capture substance is removed, and washing can be performed. The signal-generating substance may be, for example, an enzyme.

The signal-generating substance is, for example, a chromogenic, fluorescent, or chemiluminescent enzyme precursor, which is converted into a detectable label upon contact with a substrate solution.

The signal-generating substance serves to generate a signal of a detectable indicator, such as a fluorescence signal, by reacting with a substrate solution, and the enzyme component may include β-galactosidase, horseradish peroxidase, or alkaline phosphatase.

In steps 1230 and 1240, the device for detecting particles of the analyte washes the microparticles in the same manner as in step 1220.

In step 1250, the device for detecting particles of the analyte injects a substrate solution, which reacts with the signal-generating substance to generate a signal, into the reaction chamber 205 and mixes them. It is preferable to dry the microparticles remaining in the reaction chamber 205 and then mix them with the substrate solution.

The substrate solution reacts with the signal-generating substance to generate a signal of a detectable indicator, such as a fluorescence signal, and may include Resorufin β-D-galactopyranoside, or the like.

In step 1260, the device for detecting particles of the analyte injects the mixed substrate solution through the injection port 301, and captures magnetic particles in the mixed substrate solution in the microchambers 31 within the detection space 331 using the magnetic body 15.

In order to isolate the substances in the microchambers 31 arranged within the detection space 331 of the analysis chip 301 so that they do not communicate with each other, a fluid with properties that do not mix with the substrate solution may be injected, the fluid preferably including oil or air. It is preferable that the microchamber be larger than the size of the microparticle and have a size that allows at most one magnetic particle to be accommodated.

According to another exemplary embodiment of the present invention, instead of reacting the microparticles with the second capture substance in the reaction chamber 200, the first capture substance immobilized on the microparticles may bind to the analyte, and then the microparticles may be injected through the injection port 311 so that the microparticles are captured in the respective microchambers 31.

Thereafter, when the second capture substance 61 that binds to the analyte and the signal-generating substance is injected through the injection port 311, the second capture substance 61 binds to the analyte 51 bound to the first capture substance 41, and as a next step, when the signal-generating substance 71 is injected through the injection port 311, the signal-generating substance 71 binds to the second capture substance 61.

In addition, a substrate solution may be injected through the injection port 311 to cause the substrate solution to react with the signal-generating substance 71, thereby generating a signal of a detectable indicator, such as a fluorescence signal, and a hydrophobic solvent may be caused to flow through the injection port 311 to remove the remaining substrate solution after the reaction with the signal-generating substance 71.

In step 1270, the device for detecting particles of the analyte counts and detects the number of microchambers 31 in which a signal is generated due to the mixing of the signal-generating substance and the substrate solution.

As an exemplary embodiment, a first count value is calculated by recognizing and counting the microchambers in which the magnetic particles are accommodated, and a second count value is calculated by recognizing and counting the microchambers in which the signal is generated due to the mixing of the signal-generating substance and the substrate solution.

An amount of the analyte in the fluid sample may be estimated by calculating a ratio of the second count value to the first count value.

As another exemplary embodiment, a region including the plurality of microchambers may be photographed using a microscope, a digital camera, or the like, a first count value may be calculated by distinguishing the microchambers in which the magnetic particles are accommodated through image processing and counting the distinguished microchambers, and a second count value may be calculated by distinguishing the microchambers in which a signal of a detectable indicator, such as a fluorescence signal, is generated through image processing and counting the number of the distinguished microchambers.

As an exemplary embodiment of estimating the amount of particles of the analyte, the amount may be estimated by calculating a ratio of the number of microchambers in which the fluorescence signal is generated to the number of microchambers in which the magnetic particles are accommodated, and the greater the amount of the analyte in the fluid sample, the higher the ratio, and the smaller the amount of the analyte, the lower the ratio.

As another exemplary embodiment of estimating the amount of particles of the analyte, the device for detecting particles of an analyte according to an exemplary embodiment of the present invention may include three or more standard substances with known concentrations in a cartridge, generate a calibration curve showing the concentrations of the standard substances, and then correct the concentration calculation of the analyte using the generated calibration curve.

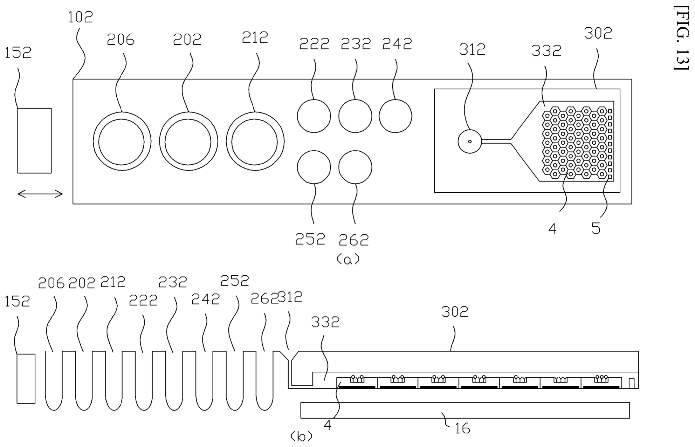

FIG. 13 is a schematic diagram of a device for detecting particles of an analyte present in trace amounts in a fluid sample according to a third exemplary embodiment of the present invention.

FIG. 13(a) is a plan view of a device for detecting particles of an analyte according to a third exemplary embodiment of the present invention, and FIG. 13(b) is a cross-sectional view of the device for detecting particles of an analyte according to the third exemplary embodiment of the present invention.

Referring to FIG. 13, the device for detecting particles of an analyte present in trace amounts in a fluid sample according to the third exemplary embodiment of the present invention includes a magnetic device 152 and a cartridge 102.

The magnetic device 152 moves closer to or farther away from a reaction chamber 206, thereby causing separation between microwell microparticles 4 within the reaction chamber 206 and a residual solution. Thereafter, after removing the residual solution, a washing solution accommodated in a washing solution chamber 232 may be injected into the reaction chamber 206 to wash a wall surface of the reaction chamber 206 where the microwell microparticles 4 are located. A magnetic material is preferably formed at a lower portion of the microwell microparticle 4.

The cartridge 102 includes a fluid sample chamber 202, the reaction chamber 206, a waste chamber 212, a microwell microparticle chamber 222, the washing solution chamber 232, a second capture substance chamber 242, a signal-generating substance chamber 252, a substrate solution chamber 262, and an analysis chip 302.

The cartridge 102 is preferably sealed with an air-impermeable film using thermal welding, UV bonding, ultrasonic welding, tape, or the like in order to prevent contamination of the plurality of chambers and the analysis chip 302.

The fluid sample chamber 202 is configured to accommodate a predetermined amount of fluid sample. The fluid sample chamber 202 may be configured to have a capacity within a range of 50 μL to 10 mL. The fluid sample includes body fluids such as blood, plasma, serum, and cerebrospinal fluid.

The reaction chamber 206 is configured to accommodate and mix a predetermined amount of a fluid sample and microwell microparticles with a first capture substance immobilized therein. The reaction chamber 206 may be configured to have a capacity within a range of 50 μL to 15 mL.

The waste chamber 212 is configured to provide a storage space for accommodating waste that is generated when a fluid sample, microwell microparticles, a washing solution, a second capture substance, a signal-generating substance, a substrate solution, and the like introduced into the analysis chip 302 are discharged. The waste chamber 212 may be configured to have a capacity within a range of 1 to 15 mL. The waste chamber 212 of FIG. 13 is included in the cartridge 102. However, the waste chamber 212 may be provided in a separate space outside the cartridge 102.

The microwell microparticle chamber 222 is configured to accommodate microwell microparticles with a first capture substance immobilized therein, and the microwell microparticles are injected into the reaction chamber 206.

It is preferable that the microwell microparticles accommodated in the microwell microparticle chamber 222 have a first capture substance bound to their surfaces, the first capture substance binding to an analyte. The first capture substance may include an antibody, a nucleic acid molecule, a peptide, or the like.

The washing solution chamber 232 is configured to accommodate a washing solution for washing a wall surface of the reaction chamber 206 and a detection space 332 of the analysis chip 302. The washing solution may be a solution such as phosphate buffered saline (PBS), tris-buffered saline (TBS), PBS-T, TBS-T, or ultrapure water.

As an example, the washing solution of the washing solution chamber 232 may be injected into an injection port 312 to wash away the fluid sample, the microparticles, the second capture substance, the signal-generating substance, the substrate solution, and the like remaining in the detection space 332 after reaction.

In another exemplary embodiment, when the magnetic device 152 comes close to the reaction chamber 205 and separation occurs between the microwell microparticles within the reaction chamber 205 and the residual solution, after removing the residual solution, the washing solution accommodated in the washing solution chamber 232 may be injected into the reaction chamber 205 to wash the wall surface of the reaction chamber 205 where the microwell microparticles are located. In this case, a magnetic material is preferably formed at a lower portion of the microwell microparticle.

The second capture substance chamber 242 is configured to accommodate a second capture substance that specifically binds to an analyte and also binds to a signal-generating substance. The second capture substance has the characteristic of specifically binding to each of the analyte and the signal-generating substance, and functions to cause the signal-generating substance to bind to the second capture substance that has specifically bound to the analyte, thereby generating a fluorescence signal proportional to the amount of the analyte. The second capture substance may be, for example, a secondary antibody and may be provided in a form pre-bound to the signal-generating substance.

Since the second capture substance binds to the analyte, and the first capture substance immobilized on the microwell microparticles also binds to the analyte, it is preferable that the first capture substance and the second capture substance bind to different sites of the analyte.

The signal-generating substance chamber 252 is configured to accommodate a signal-generating substance that specifically binds to the second capture substance bound to the analyte. The signal-generating substance may be, for example, an enzyme.

The signal-generating substance is, for example, a chromogenic, fluorescent, or chemiluminescent enzyme precursor, which is converted into a detectable label upon contact with a substrate solution.

The signal-generating substance and the second capture substance may be accommodated in a bound state in either the second capture substance chamber 242 or the signal-generating substance chamber 252.

The signal-generating substance serves to generate a signal of a detectable indicator, such as a fluorescence signal, by reacting with a substrate solution, and the enzyme component may include β-galactosidase, horseradish peroxidase, or alkaline phosphatase.

The substrate solution chamber 262 is configured to accommodate a substrate solution that reacts with the signal-generating substance to generate a signal. The substrate solution reacts with the signal-generating substance to generate a signal of a detectable indicator, such as a fluorescence signal, and may include Resorufin β-D-galactopyranoside, or the like.

The analysis chip 302 includes the injection port 312 and the detection space 332.

Since a predetermined amount of fluid sample and the microwell microparticles with the first capture substance immobilized therein are accommodated and mixed in the reaction chamber 206, the analyte is bound to the first capture substance immobilized on the microwell microparticles.

The fluid sample, the microwell microparticles, the washing solution, the second capture substance, the signal-generating substance, and the substrate solution, or a mixture is injected through the injection port 312.

The injection port 312 may further include a filter to separate and supply particles larger than microwell microparticles from a mixed solution of the microwell microparticles and the fluid sample. The filter may be formed of various materials with pores of various sizes to filter out biological cells, inorganic particles, organic particles, or the like. It is preferable that the filter of the third exemplary embodiment be formed with pores capable of filtering out particles larger than the microwell microparticles. Additionally, the filter may be formed of a biologically inert material so that it can be applied to biological samples.

In the process of supplying the fluid sample to the injection port 312, an output surface of the filter may be pre-wetted by wetting it with a buffer solution before filtration. Therefore, the mixed solution of microwell microparticles and fluid sample supplied to an inlet surface of the filter can pass through the filter pores without surface resistance and be smoothly filtered to the outlet surface of the filter.

When the mixed solution of microwell microparticles and the fluid sample is injected into the detection space 332 through the injection port 312, the microwell microparticles are arranged in the inner space within the detection space 332. By placing a magnetic body 16 below the microwell microparticles 4, the microwell microparticles can be easily immobilized in the inner space of the detection space 332.

Thereafter, when the washing solution is injected through the injection port 312, the microwell microparticles not immobilized in the inner space of the detection space 332, the first capture substance not immobilized on the microwell microparticles 4, and the fluid sample not bound to the first capture substance can be removed, and washing can be performed.

Note that, when the second capture substance accommodated in the second capture substance chamber 242 that binds to the analyte and the signal-generating substance is injected through the injection port 312, the second capture substance binds to the analyte bound to the first capture substance. Subsequently, the remaining second capture substance can be removed, and washing can be performed by the washing solution.

Next, when a signal-generating substance is injected through the injection port 312, the signal-generating substance binds to the second capture substance.

Finally, a substrate solution is injected through the injection port 312 to cause the substrate solution to react with the signal-generating substance, thereby generating a signal of a detectable indicator, such as a fluorescence signal, and a hydrophobic solvent is caused to flow through the injection port 312 to remove the remaining substrate solution after the reaction with the signal-generating substance. The hydrophobic solvent is preferably oil or gas.

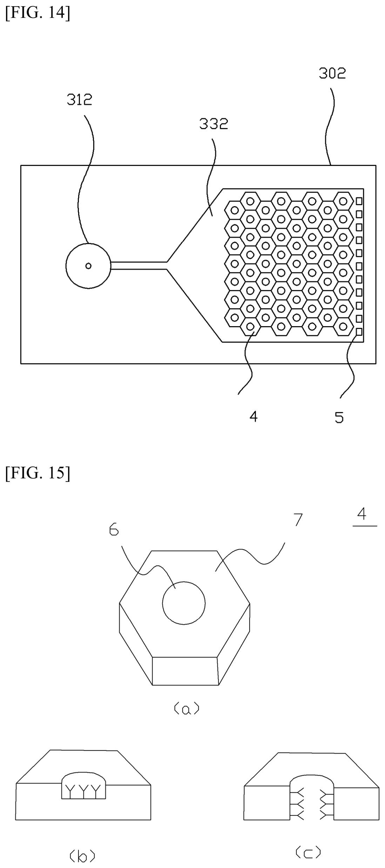

FIG. 14 is a diagram illustrating in more detail an analysis chip 302 according to the third exemplary embodiment of the present invention.

The mixed solution of fluid sample and microwell microparticles, the washing solution, the second capture substance, the signal-generating substance, and the substrate solution, or a mixed solution thereof are successively injected through the injection port 312. The second capture substance is a capture substance for detecting a signal of a detectable indicator, such as a fluorescence signal, and may be provided with bound to the signal-generating substance, which can simplify the procedure compared to when the second capture substance and the signal-generating substance are provided separately.

When the mixed solution of the fluid sample and the microwell microparticles 4, the washing solution, the second capture substance, the signal-generating substance, and the substrate solution or the mixed solution thereof are injected through the injection port 312, they diffuse and flow into the detection space 332, and pillars 5 are formed at one end of the detection space 332 so that the microwell microparticles 4 can collect into the detection space 332.

As illustrated in FIG. 14, the microwell microparticles 4 are collected in the detection space 302, and include a mix of microwell microparticles where the first capture substance immobilized in the inner spaces of the microwell microparticles 4 and the analyte in the fluid sample are bound and microwell microparticles where they are not bound.

FIG. 15 is a diagram illustrating in more detail a microwell microparticle according to the third exemplary embodiment of the present invention.

Referring to FIG. 15, the microwell microparticle 4 has a hexagonal upper surface, and a receiving portion 6, which is the inner space of the microwell microparticle 4, may be open on one surface, or may have a through shape with both surfaces open in a donut form.

The receiving portion 6 of the microwell microparticle 4 may have a cylindrical shape with one surface closed (FIG. 15(b)) or a cylindrical shape with both surfaces open (FIG. 15(c)).

Note that the first capture substance may be immobilized in the receiving portion 6 of the microwell microparticle 4 and may bind to an analyte.

The receiving portion 6, which is the inner space of the microwell microparticle 4, and the first capture substance, are coated with a hydrophilic solvent, and an outer space 7 of the microwell microparticle 4 is coated with a hydrophobic solvent, so that, when a hydrophobic solvent is caused to flow over the substrate, a substrate solution in the outer space 7 of the microwell microparticle 4 can be removed.

Comparing FIG. 7 and FIG. 15, the difference is that in FIG. 15, the first capture substance is immobilized in the inner space of the microwell microparticle 4, whereas in FIG. 7, the first capture substance is immobilized on the microparticle.

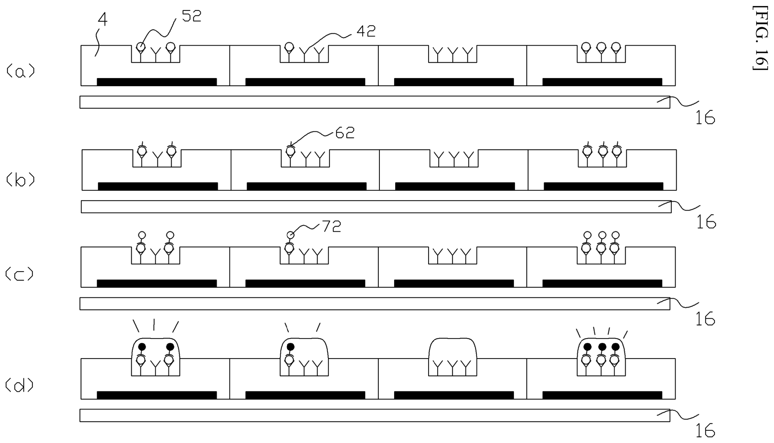

FIG. 16 is a conceptual diagram illustrating a process for detecting an analyte according to the third exemplary embodiment of the present invention.

The mixed solution of fluid sample and microwell microparticles 4, the washing solution, the second capture substance, the signal-generating substance, and the substrate solution, or a mixed solution thereof are successively injected through the injection port 312.

FIG. 16(a) illustrates a state in which the microwell microparticles 4 with the first capture substance 42 immobilized therein, after being mixed and reacted with a fluid sample in the reaction chamber 206 and injected through the injection port 312, are arranged and immobilized by the magnetic body 16. A first capture substance 42 is immobilized in the microwell microparticles 4 to specifically capture an analyte 52.

The fluid sample and the microwell microparticles 4 may be mixed in the reaction chamber 206. In the inner space of the microwell microparticle 4, the first capture substance is immobilized, and the analyte in the fluid sample is bound to the first capture substance.

When the mixed solution of the fluid sample and the microwell microparticles 4 is injected through the injection port 312, it diffuses and flows into the detection space 332. The mixed solution of the fluid sample and the microwell microparticles 4 that has passed through the detection space 332 is blocked by the plurality of pillars 5 formed on an end opposite to the injection port 312 and is collected in the detection space 332.

Thereafter, when the washing solution is injected through the injection port 312, the fluid sample containing the analyte that has not been captured by the binding between the first capture substance and the analyte or by the first capture substance can be removed, and washing can be performed.

As can be seen in FIG. 16(b), when the second capture substance 62 accommodated in the second capture substance chamber 242 that binds to the analyte and the signal-generating substance is injected through the injection port 312, the second capture substance 62 binds to the analyte 52 bound to the first capture substance 42. Subsequently, the remaining second capture substance 62 can be removed, and washing can be performed by the washing solution.

In FIG. 16(c), when the signal-generating substance 72 is injected through the injection port 312, the signal-generating substance 72 binds to the second capture substance 62. It is preferable that the remaining signal-generating substance 72 be then washed away by a washing solution.

In FIG. 16(d), a substrate solution is injected through the injection port 312 to cause the substrate solution to react with the signal-generating substance 72, thereby generating a signal of a detectable indicator, such as a fluorescence signal, and a hydrophobic solvent is caused to flow through the injection port 312 to remove the remaining substrate solution after the reaction with the signal-generating substance 72.

Then, a hydrophobic solvent is caused to flow over the substrate to remove the substrate solution present outside the microwell microparticles 4, and the number of microwell microparticles 4 where a signal of a detectable indicator, such as a fluorescence signal, is generated is counted and detected. The inside 6 of the microwell microparticle 4 is coated with a hydrophilic solvent, and the outside 7 of the microwell microparticle 4 is coated with a hydrophobic solvent, so that, when the hydrophobic solvent is caused to flow over the substrate, the substrate solution present on the outside 7 of the microwell microparticle 4 can be easily removed.

For more accurate detection of the analyte contained in the fluid sample, the number of particles of the analyte may be correlated with the brightness of a signal of a detectable indicator, such as a fluorescence signal, and the number of particles of the analyte may be counted by considering the brightness of the fluorescence signal of each microwell microparticle 4.

Referring again to FIG. 16(d), it can be seen that the number of signal-generating substances that generate a signal of a detectable indicator signal, such as a fluorescence signal, differs from microwell microparticle 4 to microwell microparticle 4, and the brightness of the fluorescence signal is different accordingly.

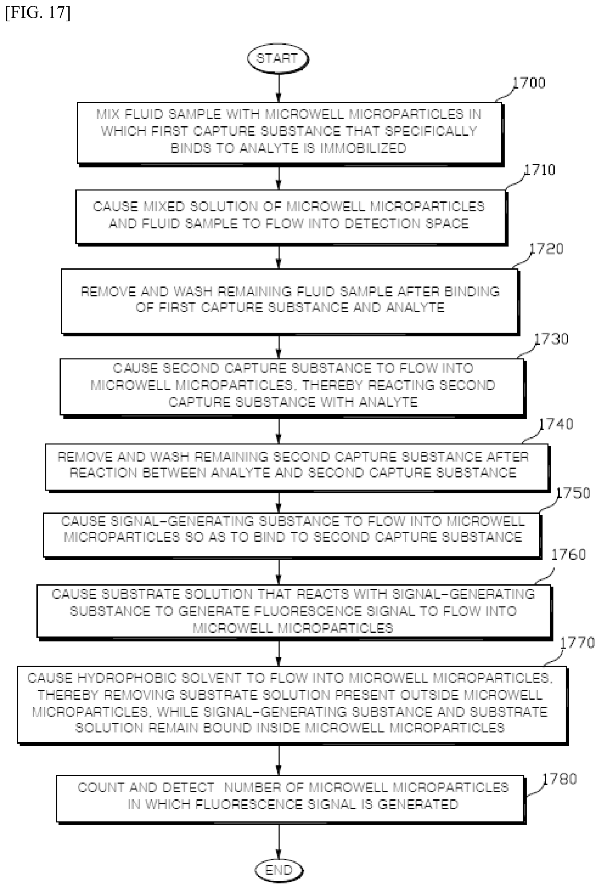

FIG. 17 is a flowchart of a method for detecting particles of an analyte present in trace amounts in a fluid sample according to the third exemplary embodiment of the present invention.

In step 1700, the fluid sample is mixed with the microwell microparticles 4 in which the first capture substance that specifically binds to the analyte is immobilized, in the reaction chamber 206.

The mixed fluid sample and microwell microparticles 4 are injected through the injection port 312, and the first capture substance 42 is immobilized in the inner spaces 6 of the microwell microparticles 4.

In step 1710, the mixed solution of the microwell microparticles 4 and the fluid sample is caused to flow into the detection space 332. The microwell microparticles 4 flow over the detection space 332 with the analyte 52 in the fluid sample bound to the first capture substance 42.

In step 1720, the remaining fluid sample after the binding of the first capture substance 42 and the analyte 52 is removed, and washing can be performed.

In step 1730, the second capture substance 62 that specifically binds to the analyte 52 and binds to the signal-generating substance 72 is caused to flow into the microwell microparticles 4, thereby reacting the second capture substance 62 with the analyte 52.

In step 1740, the remaining second capture substance 62 after the reaction between the analyte 52 and the second capture substance 62 is removed and washed.

In step 1750, the signal-generating substance 72 is caused to flow into the microwell microparticles 4 so as to bind to the second capture substance 62.

In step 1760, a substrate solution that reacts with the signal-generating substance 72 to generate a signal of a detectable indicator, such as a fluorescence signal, is caused to flow into the microwell microparticles 4.

In step 1770, a hydrophobic solvent is caused to flow into the microwell microparticles 4, thereby removing the substrate solution present outside the microwell microparticles 4, while the signal-generating substance 72 and the substrate solution remain bound inside the microwell microparticles 4.

It is preferable that the inside 6 of the microwell microparticle 4 be hydrophilic, and the outside 7 of the microwell microparticle 4 be hydrophobic.

In step 1780, the number of microwell microparticles 4 in which the signal of the detectable indicator, such as the fluorescence signal, is generated is counted and detected.

As an exemplary embodiment, a first count value is calculated by recognizing and counting the microwell microparticles 4, and a second count value is calculated by recognizing and counting the microwell microparticles 4 in which the signal is mixed due to the mixing of the signal-generating substance and the substrate solution.

An amount of the analyte in the fluid sample may be estimated by calculating a ratio of the second count value to the first count value.

As another exemplary embodiment, a region including the microwell microparticles 4 may be photographed using a microscope, a digital camera, or the like, a first count value may be calculated by distinguishing the microwell microparticles 4 through image processing and counting the distinguished microwell microparticles 4, and a second count value may be calculated by distinguishing the microwell microparticles 4 in which the fluorescence signal is generated through image processing and counting the number of the distinguished microwell microparticles 4.

As an exemplary embodiment of estimating the amount of particles of the analyte, the amount may be estimated by calculating a ratio of the number of microwell microparticles 4 generating a signal of a detectable indicator, such as a fluorescence signal, to the total number of microwell microparticles 4, and the greater the amount of the analyte in the fluid sample, the higher the ratio, and the smaller the amount of the analyte, the lower the ratio.

As another exemplary embodiment of estimating the amount of particles of the analyte, the device for detecting particles of an analyte according to an exemplary embodiment of the present invention may include three or more standard substances with known concentrations in a cartridge, generate a calibration curve showing the concentrations of the standard substances, and then correct the concentration calculation of the analyte using the generated calibration curve.

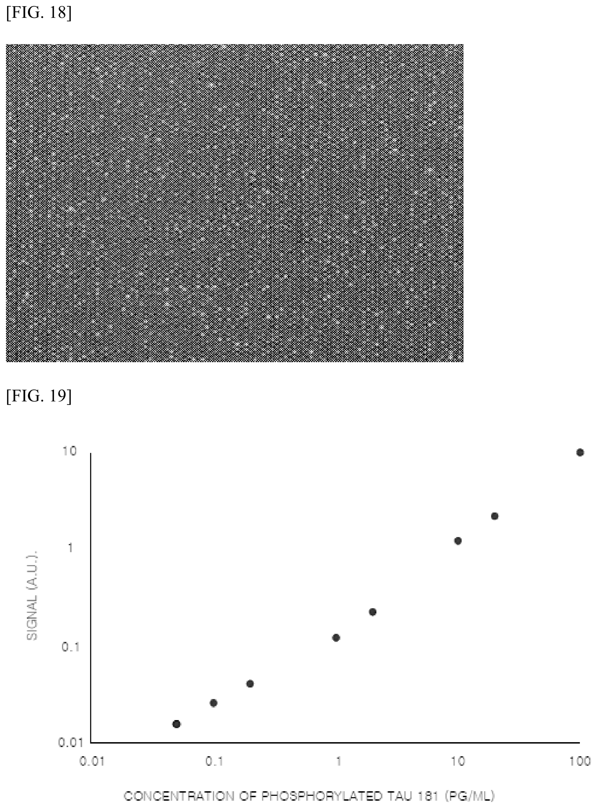

FIG. 18 is a diagram showing microchambers including microparticles and microchambers generating a fluorescence signal, according to the second exemplary embodiment of the present invention.

A microchamber including a microparticle to which an analyte is not bound is represented as a dark cell, and a microchamber generating a fluorescence signal is represented as a bright cell.

Instead of counting the number of cells to calculate the ratio of the number of microchambers including microparticles to which an analyte is not bound to the number of microchambers generating the fluorescence signal, the ratio of the second count value to the first count value may be calculated by collecting bright cells and calculating the area of the bright cell region or by collecting dark cells and calculating the area of the dark cell region, thereby estimating the amount of the analyte in the fluid sample.

When micro-beads (small, bead-like proteins) coated with an antibody as the first capture substance are the microparticles according to the second exemplary embodiment of the present invention, a bead in which a signal is generated by a second capture substance bound to a desired protein (an analyte) in a serum or plasma sample that has been captured by the first capture substance, and by an enzyme (signal-generating substance) bound to the second capture substance, may be referred to as an "on" bead.

When determining the concentration of the analyte according to the second exemplary embodiment of the present invention, the concentration of the enzyme (AEB) is determined using a digital and analog analysis system, and the concentration is preferably determined by the number of "on" beads (activated proteins).

The concentration of the desired protein (analyte) in a serum or plasma sample may be obtained by converting the proportion of active beads into the concentration of enzyme (AEB) using a Poisson distribution when the ratio of enzyme (signal-generating substance) to beads (microparticles) is low or when there are no beads that generate a signal by the enzyme.

The Poisson distribution can describe the likelihood of multiple events occurring when the mean number of events is known. If the expected mean number of occurrences is $\mu$, the probability that exactly v occurrences can be expressed as the following mathematical expression 1 (v is a non-negative integer, $v=0, 1, 2, 3, \cdots$).

[Mathematical Equation 1]

$$P_{\mu}(v) = e^{-\mu}\left(\frac{\mu^{v}}{v!}\right)$$

$\mu$ is equal to the proportion of proteins bound to the beads, and v is equal to the number of enzyme-labeled proteins carried by each subpopulation of beads (i.e., 0, 1, 2, 3, etc.).

According to the second exemplary embodiment of the present invention, $\mu$, which is equal to the concentration of the enzyme (AEB), may be determined and used as a quantitative parameter to determine the protein concentration.

A bead associated with one enzyme is indistinguishable from a bead associated with two enzymes, where the population of beads associated with one enzyme exhibits a distribution of up to sevenfold during the activation step. Due to this wide distribution, a portion of the "off" beads (inactivated proteins) can be identified when $v=0$ ($P_{\mu}(0)$).

By determining $P_{\mu}(0)$ using Mathematical Equation 1, and using the fact that the proportion of "off" beads is equal to 1 minus the proportion of "on" beads, $AEB_{digital}$ (AEB determined digitally) can be determined from $f_{on}$ (the proportion or % activity of "on" beads).

[Mathematical Equation 2]

$$\mu = AEB_{digital} = -\ln[1 - f_{on}]$$

The concentration of the protein (analyte) in a plasma sample can be accurately measured through Mathematical Equation 2.

Concentration quantification using the Poisson distribution is reliably measured when the activated beads are less than 70%, but has a limitation in that when the proportion of activated beads exceeds approximately 70%, the change in AEB with concentration decreases, leading to increased inaccuracy in concentration measurement.

To overcome this, AEB can be determined from the average fluorescence intensity value of the activated beads ($\bar{I}_{bead}$) and the average fluorescence intensity generated by a single enzyme ($\bar{I}_{single}$). The AEB of an array in the analog range ($AEB_{analog}$) is defined as in Mathematical Equation 3.

[Mathematical Equation 3]

$$AEB_{analog} = \frac{f_{on} \times \bar{I}_{bead}}{\bar{I}_{single}}$$

To determine the fluorescence intensity of $\bar{I}_{single}$, it is desirable to equate the AEB values of Mathematical Equations 2 and 3 at the proportion of activated beads.

[Mathematical Equation 4]

$$\bar{I}_{single} = \frac{f_{on} \times \bar{I}_{bead}}{-\ln[1-f_{on}]}, \text{ in arrays where } f_{on} < 0.1$$

In this case, it is preferable to select an array of "on" beads < 0.1 that satisfies the criterion of Mathematical Equation 4.

Therefore, the concentration of the enzyme (AEB) can be configured for both digital and analog ranges.

FIG. 19 is a graph showing the relationship between the concentration of phosphorylated tau 181 (pg/mL) and the signal (a.u.).

Phosphorylated tau 181 protein is a protein used in the diagnosis of Alzheimer's disease and corresponds to the analyte of the present invention.

A second capture substance binds to the phosphorylated tau 181 protein, and an enzyme (signal-generating substance) binds to the second capture substance. Then, a signal is generated by a substrate solution. FIG. 19 shows the relationship between the concentration of phosphorylated tau 181 protein and the signal generation intensity.

Referring to FIG. 19, it can be confirmed that the concentration of phosphorylated tau 181 protein and the signal generation intensity are proportional.

Hereinabove, although the present invention has been described by specific matters such as specific components, the non-limiting exemplary embodiments, and the drawings, they have been provided only for assisting in the more general understanding of the present invention. Therefore, the present invention is not limited to the exemplary embodiments, and various modifications and changes can be made by one skilled in the art to which the present invention belongs from the above description.

Therefore, the spirit of the present invention should not be limited to the aforementioned exemplary embodiments, and the following claims as well as all modifications equal or equivalent to the claims are intended to fall within the scope and spirit of the present invention.

**Claims**

1. A method for detecting an analyte present in trace amounts in a fluid sample, the method comprising:

    causing a fluid sample containing an analyte to flow over a substrate having an array of microchambers formed on a surface thereof, with a first capture substance that specifically binds to the analyte immobilized in the microchambers;
    binding the analyte to the first capture substance in each microchamber of the array of microchambers;
    causing a second capture substance, which specifically binds to the analyte and binds to a signal-generating substance, to flow over the substrate to react the analyte with the second capture substance;
    causing the signal-generating substance to flow over the substrate to bind to the second capture substance;
    causing a substrate solution, which reacts with the signal-generating substance to generate a fluorescence signal, to flow over the substrate;
    causing a hydrophobic solvent to flow over the substrate to remove the substrate solution present outside the microchambers when the signal-generating substance and the substrate solution react in the microchambers; and
    counting and detecting the number of microchambers in which the fluorescence signal is generated.

2. The method of claim 1, wherein the inside of the microchamber with the first capture substance immobilized therein is hydrophilic, and the outside of the microchamber is hydrophobic.

3. The method of claim 1, further comprising calculating a first count value by counting the microchambers, calculating a second count value by recognizing and counting the microchambers in which a signal is generated due to mixing of the signal-generating substance and the substrate solution, and estimating an amount of the analyte in the fluid sample by calculating a ratio of the second count value to the first count value.

4. The method of claim 1, further comprising generating a calibration curve showing a concentration of a standard substance with a known concentration for the fluid sample, and then correcting a concentration calculation of the analyte using the generated calibration curve.

5. The method of claim 1, further comprising estimating an amount of the analyte in the fluid sample by calculating a first count value by counting the microchambers, and calculating a second count value by capturing an image of a region of the microchambers, distinguishing the microchambers in which the fluorescence signal is generated through image processing, and counting the number of the distinguished microchambers.

[FIG. 1]

[FIG. 2]

[FIG. 3]

[FIG. 4]

START

CAUSE FLUID SAMPLE TO FLOW OVER SUBSTRATE HAVING ARRAY OF MICROCHAMBERS FORMED ON SURFACE THEREOF, WITH CAPTURE SUBSTANCE THAT SPECIFICALLY BINDS TO ANALYTE IMMOBILIZED IN MICROCHAMBERS — 400

BIND ANALYTE TO CAPTURE SUBSTANCE IN EACH MICROCHAMBER OF ARRAY OF MICROCHAMBERS — 410

REMOVE AND WASH REMAINING FLUID SAMPLE AFTER BINDING OF CAPTURE SUBSTANCE AND ANALYTE — 420

CAUSE SECOND CAPTURE SUBSTANCE, WHICH SPECIFICALLY BINDS TO ANALYTE AND BINDS TO SIGNAL-GENERATING SUBSTANCE, TO FLOW OVER SUBSTRATE TO REACT ANALYTE WITH SECOND CAPTURE SUBSTANCE — 430

REMOVE AND WASH REMAINING SECOND CAPTURE SUBSTANCE AFTER REACTION BETWEEN ANALYTE AND SECOND CAPTURE SUBSTANCE — 440

CAUSE SIGNAL-GENERATING SUBSTANCE TO FLOW OVER SUBSTRATE TO BIND TO SECOND CAPTURE SUBSTANCE — 450

CAUSE SUBSTRATE SOLUTION, WHICH REACTS WITH SIGNAL-GENERATING SUBSTANCE TO GENERATE FLUORESCENCE SIGNAL, TO FLOW OVER SUBSTRATE — 460

CAUSE HYDROPHOBIC SOLVENT TO FLOW OVER SUBSTRATE TO REMOVE SUBSTRATE SOLUTION PRESENT OUTSIDE MICROCHAMBERS, WHILE SIGNAL-GENERATING SUBSTANCE AND SUBSTRATE SOLUTION REMAIN BOUND IN MICROCHAMBERS — 470

COUNT AND DETECT NUMBER OF MICROCHAMBERS IN WHICH FLUORESCENCE SIGNAL IS GENERATED — 480

END

[FIG. 5]

[FIG. 6]

[FIG. 7]

(a)          (b)

[FIG. 8]

[FIG. 9]

EP 4 697 009 A1

26

[FIG. 10]

(a)

(b)

[FIG. 11]

(a)

(b)

[FIG. 12]

START

MIX FIRST CAPTURE SUBSTANCE WITH MICROPARTICLES TO
IMMOBILIZE FIRST CAPTURE SUBSTANCE ON MICROPARTICLES — 1200

REACT FIRST CAPTURE SUBSTANCE IMMOBILIZED ON MICROPARTICLES
WITH ANALYTE IN FLUID SAMPLE, IN REACTION CHAMBER — 1210

BRING MAGNETIC DEVICE CLOSE TO REACTION CHAMBER TO SEPARATE
MICROPARTICLES FROM FLUID SAMPLE, REMOVE AND WASH FLUID SAMPLE NOT
BOUND TO MICROPARTICLES — 1220

INJECT SECOND CAPTURE SUBSTANCE THAT SPECIFICALLY BINDS TO
ANALYTE BOUND TO FIRST CAPTURE SUBSTANCE INTO REACTION
CHAMBER AND REACT IT THEREIN — 1230

INJECT SIGNAL−GENERATING SUBSTANCE THAT SPECIFICALLY BINDS TO SECOND
CAPTURE SUBSTANCE INTO REACTION CHAMBER AND REACT IT THEREIN — 1240

INJECT SUBSTRATE SOLUTION THAT REACTS WITH SIGNAL−GENERATING
SUBSTANCE TO GENERATE SIGNAL INTO REACTION CHAMBER AND MIX THEM — 1250

INJECT MIXED SUBSTRATE SOLUTION THROUGH INJECTION PORT, AND
CAPTURE MAGNETIC PARTICLES IN SUBSTRATE SOLUTION IN
MICROCHAMBERS WITHIN DETECTION SPACE USING MAGNETIC BODY — 1260

COUNT AND DETECT NUMBER OF MICROCHAMBERS IN WHICH SIGNAL IS GENERATED
BY MIXING OF SIGNAL−GENERATING SUBSTANCE AND SUBSTRATE SOLUTION — 1270

END

[FIG. 13]

[FIG. 14]

[FIG. 15]

(a)

(b)                    (c)

[FIG. 16]

[FIG. 17]

START

MIX FLUID SAMPLE WITH MICROWELL MICROPARTICLES IN WHICH FIRST CAPTURE SUBSTANCE THAT SPECIFICALLY BINDS TO ANALYTE IS IMMOBILIZED — 1700

CAUSE MIXED SOLUTION OF MICROWELL MICROPARTICLES AND FLUID SAMPLE TO FLOW INTO DETECTION SPACE — 1710

REMOVE AND WASH REMAINING FLUID SAMPLE AFTER BINDING OF FIRST CAPTURE SUBSTANCE AND ANALYTE — 1720

CAUSE SECOND CAPTURE SUBSTANCE TO FLOW INTO MICROWELL MICROPARTICLES, THEREBY REACTING SECOND CAPTURE SUBSTANCE WITH ANALYTE — 1730

REMOVE AND WASH REMAINING SECOND CAPTURE SUBSTANCE AFTER REACTION BETWEEN ANALYTE AND SECOND CAPTURE SUBSTANCE — 1740

CAUSE SIGNAL-GENERATING SUBSTANCE TO FLOW INTO MICROWELL MICROPARTICLES SO AS TO BIND TO SECOND CAPTURE SUBSTANCE — 1750

CAUSE SUBSTRATE SOLUTION THAT REACTS WITH SIGNAL-GENERATING SUBSTANCE TO GENERATE FLUORESCENCE SIGNAL TO FLOW INTO MICROWELL MICROPARTICLES — 1760

CAUSE HYDROPHOBIC SOLVENT TO FLOW INTO MICROWELL MICROPARTICLES, THEREBY REMOVING SUBSTRATE SOLUTION PRESENT OUTSIDE MICROWELL MICROPARTICLES, WHILE SIGNAL-GENERATING SUBSTANCE AND SUBSTRATE SOLUTION REMAIN BOUND INSIDE MICROWELL MICROPARTICLES — 1770

COUNT AND DETECT NUMBER OF MICROWELL MICROPARTICLES IN WHICH FLUORESCENCE SIGNAL IS GENERATED — 1780

END

[FIG. 18]

[FIG. 19]

## INTERNATIONAL SEARCH REPORT

| International application No. |
| --- |
| **PCT/KR2023/007464** |

| A. | CLASSIFICATION OF SUBJECT MATTER |
| --- | --- |

**G01N 15/14**(2006.01)i; **G01N 15/10**(2006.01)i; **G01N 21/64**(2006.01)i; **G01N 33/487**(2006.01)i

According to International Patent Classification (IPC) or to both national classification and IPC

| B. | FIELDS SEARCHED |
| --- | --- |

Minimum documentation searched (classification system followed by classification symbols)

G01N 15/14(2006.01); C07K 16/00(2006.01); G01N 27/00(2006.01); G01N 27/26(2006.01); G01N 27/327(2006.01); G01N 27/48(2006.01); G01N 33/48(2006.01); G01N 33/53(2006.01); G01N 33/543(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 챔버(chamber), 유체(fluid), 기판(substrate), 캘리브레이션(calibration), 입자 (particle)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2009-0060143 A (ELECTRONICS AND TELECOMMUNICATIONS RESEARCH INSTITUTE) 11 June 2009 (2009-06-11) See paragraphs [0029]-[0032]; and figures 1-2. | 1-5 |
| A | JP 2022-053276 A (JVC KENWOOD CO.) 05 April 2022 (2022-04-05) See entire document. | 1-5 |
| A | KR 10-2420459 B1 (BIOMETRIX TECHNOLOGY INC.) 14 July 2022 (2022-07-14) See entire document. | 1-5 |
| A | JP 2004-512496 A (CENTRE NATIONAL DE LA RECHERCHE SCI-ENTIFIQUE (CNRS)) 22 April 2004 (2004-04-22) See entire document. | 1-5 |
| A | KR 10-2012-0091872 A (LG ELECTRONICS INC.) 20 August 2012 (2012-08-20) See entire document. | 1-5 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **08 January 2024** | **09 January 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/007464**

| Patent document cited in search report | | | Publication date (day/month/year) | Patent family member(s) | | | Publication date (day/month/year) |
|---|---|---|---|---|---|---|---|
| KR | 10-2009-0060143 | A | 11 June 2009 | KR | 10-1003534 | B1 | 30 December 2010 |
| JP | 2022-053276 | A | 05 April 2022 | None | | | |
| KR | 10-2420459 | B1 | 14 July 2022 | None | | | |
| JP | 2004-512496 | A | 22 April 2004 | AU | 2002-925601 | A | 08 January 2002 |
| | | | | CA | 2413929 | A1 | 03 January 2002 |
| | | | | CA | 2413929 | C | 29 December 2009 |
| | | | | DE | 60114016 | T2 | 22 June 2006 |
| | | | | EP | 1303759 | A2 | 23 April 2003 |
| | | | | EP | 1303759 | B1 | 12 October 2005 |
| | | | | FR | 2810739 | A1 | 28 December 2001 |
| | | | | FR | 2810739 | B1 | 26 January 2007 |
| | | | | JP | 4768202 | B2 | 07 September 2011 |
| | | | | US | 2003-0186274 | A1 | 02 October 2003 |
| | | | | US | 2006-0228814 | A1 | 12 October 2006 |
| | | | | US | 7045364 | B2 | 16 May 2006 |
| | | | | WO | 02-001178 | A3 | 13 February 2003 |
| KR | 10-2012-0091872 | A | 20 August 2012 | US | 2013-0323827 | A1 | 05 December 2013 |
| | | | | US | 8961883 | B2 | 24 February 2015 |
| | | | | WO | 2012-108651 | A2 | 16 August 2012 |
| | | | | WO | 2012-108651 | A3 | 20 December 2012 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110024846